# EUROPÄISCHE PATENTSCHRIFT

(11) **EP 1 984 005 B1**
(45) Veröffentlichungstag und Bekanntmachung des Hinweises auf die Patenterteilung: **06.04.2011**
(21) Anmeldenummer: 07701315.9
(22) Anmeldetag: 13.02.2007
(51) Int. Cl.: A61K 33/24, A61P 35/00

(54) **VERWENDUNG VON GALLIUM (III) KOMPLEXEN ZUR BEHANDLUNG VON MELANOMEN**
USE OF GALLIUM(III) COMPLEXES FOR THE TREATMENT OF MELANOMAS
UTILISATION DE COMPLEXES DE GALLIUM (III) POUR LE TRAITEMENT DE MÉLANOMES

(30) Priorität: 13.02.2006 AT 2202006
(43) Veröffentlichungstag der Anmeldung: 29.10.2008
(73) Patentinhaber: Niiki Pharma Inc., Hoboken, NJ 07030 (US)
(72) Erfinder: KEPPLER, Bernhard, 68766 Hockenheim (DE)
(74) Vertreter: Atkinson, Jonathan David Mark
(86) Internationale Anmeldenummer: PCT/AT2007/000071
(87) Internationale Veröffentlichungsnummer: WO 2007/092978

(56) Entgegenhaltungen:
- WO-A-93/02087
- WO-A-02/074304
- WO-A-02/081484

## Beschreibung

Die Erfindung betrifft die Verwendung von Gallium(III)-Komplexen zur Behandlung von Melanomen.

Es ist bekannt, einfache Galliumsalze wie z.B. Gallium(III)chlorid und Galliumnitrat zur Bekämpfung von Tumorerkrankungen am Menschen einzusetzen. So beschreibt Collery in US 4,596,710 die Verwendung von Galliumchlorid bei der Behandlung verschiedener humaner Tumore. In US 4,529,593 wird die Verwendung von unter anderem Galliumnitrat zur Behandlung von tumorassoziierter Hypercalcemie beschrieben. Ein schwerwiegender Nachteil dieser kleinen anorganischen Verbindungen ist einerseits die nur sehr begrenzte Bioverfügbarkeit bei oraler Applikation als auch die große Nephrotoxizität, die eine klinische Anwendung am Menschen sehr erschweren (Krakoff et al., Cancer 44, 1722-1727, 1997; Senderowicz et al., Urol. Int. 1999, 63, 120-125; Fagbemi et al. Seminars in Urologic Oncology, 1998, 16, 23-29; Schwartz et al. Anticancer Res. 1984, 4, 317-318). Daneben wurde bei der Anwendung von einfachen Galliumsalzen auch Gewichtsverlust, Pneumonie und Leberschäden beobachtet (Hart et al, J. Natl. Cancer Inst. 47, 1121-1127, 1971). Daher wurde nach Alternativen zu den einfachen Galliumsalzen Galliumchlorid und Galliumnitrat gesucht.

Eine Verbindung, die die oben genannten Nachteile vermeidet, ist das Gallium-Maltolat, das sich derzeit in der klinischen Erprobung befindet (Lawrence Bernstein, WO 93/09776) Diese Verbindung zeichnet sich durch eine signifikant erhöhte Bioverfügbarkeit aus.

Auch Gallium(III)-Komplexe mit Stickstoffhaltigen Liganden zeigen eine sehr viel höherer Lipophilie und verbesserter Bioverfügbarkeit bei oraler Applikation und konnten in experimentellen Tiertumoren wie Weichteilsarkomen ihre Antitumorwirkung zeigen (Collery et al. WO 93/02087; Thiel et al. in: Relevance of Tumour Models for Anticancer Drug Development. Contrib. Oncol. Basel, Karger, 54, 439-442, 1999). Die orale Applikation ist bei Galliumverbindungen bei der Behandlung von Tumorerkrankungen besonders wünschenswert, da diese Verbindungen aufgrund des Mechanismus Ribonukleotid-Reduktase-Inhibition nach Möglichkeit kontinuierlich über einen längeren Zeitraum gegeben werden sollten.

In der WO 02/074304 wird die Verwendung von Gallium(III)-Komplexen mit Stickstoffhaltigen Liganden in Kombination mit anderen therapeutisch wirksamen Zytostatika wie z.B. verschiedenen Platin(II)-Komplexen beschrieben.

Überraschend hat sich nunmehr herausgestellt, dass Gallium(III)-Komplexe der allgemeinen Formel (I) auch ohne die Kombination mit weiteren zytotoxischen Verbindungen besonders zur Verwendung zur Behandlung von Melanomen geeignet sind. Nach wie vor ist der Bedarf an wirksamen Arzneimitteln für diese Indikation groß. In präklinischen Untersuchungen konnte gezeigt werden, dass Gallium-Komplexe der allgemeinen Formel (I) bei der Behandlung dieser Krebserkrankungen eine große Aktivität besitzen.

Daher liegt der Erfindung die Aufgabe zugrunde, Melanome zu behandeln.

Diese Aufgabe wird gelöst durch die Verwendung einer Verbindung der allgemeinen Formel (I) 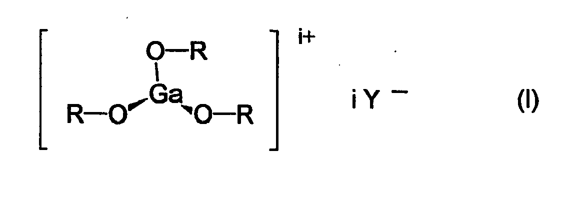 worin R
eine N-haltige Gruppe ist, ausgewählt aus einer Gruppe der allgemeinen Formel (II) bis (VII): 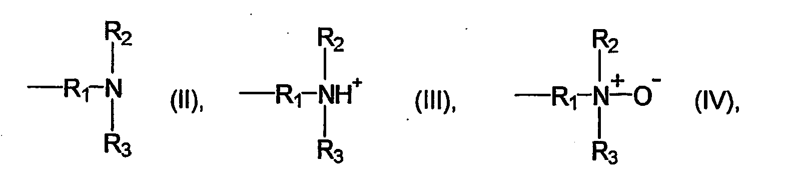 worin
R₁ C₁ - C₆ -Alkylen, C₃ - C₈ -Cycloalkylen, C₃ - C₈ - Cycloalkenylen, C₂ - C₆ -Alkenylen, ein ein- oder mehrkerniges gegebenenfalls aromatisches C₆ - C₁₄ -Ringsystem oder ein Heterocyclus, die jeweils substituiert oder unsubstituiert sein können, ist;
R₂ und R₃ C₁ - C₁₀ Alkyl, C₃ - C₈- Cycloalkyl, C₃ - C₈-Cycloalkenyl, C₂ - C₁₀-Alkenyl, ein ein- oder mehrkemiges gegebenenfalls aromatisches C₆ - C₁₄ -Ringsystem, oder ein Heterocyclus, die jeweils substituiert oder unsubstituiert sein können, oder Wasserstoff ist;
und R₁ und R₂, oder R₁ und R₃, oder R₂ und R₃ einen Heterocyclus bilden können, der gegebenenfalls weitere Stickstoffatome enthalten kann; 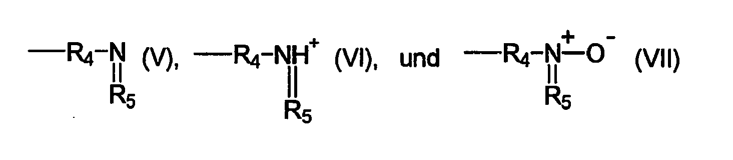 worin
R₄ die gleiche Bedeutung wie R₁ besitzt, R₅ die gleiche Bedeutung wie R₂ besitzt, und
R₄ und R₅ zusammen mit N ein gegebenenfalls aromatisches Ringsystem bilden können, das weitere Stickstoffatome enthalten kann;
- i: eine ganze Zahl von 0 bis 3 ist und der Summe der N-haltigen Gruppen der Formeln (III) und/oder (VI) entspricht;

- Y: ein Halogen, Pseudohalogen, HCO₃ oder R'COO, worin R' C₁ - C₆ -Alkyl, C₂ - C₆ -Alkenyl, C₃ - C₆ -Cycloalkyl, C₃ - C₆ - Cycloalkenyl, Aryl, die jeweils substituiert oder unsubstituiert sein können, und/oder ein physiologisch veträgliches Anion ist; und
physiologisch verträgliche Additionssalze davon,
Weiterhin ist es bevorzugt, dass der Heterocyclus für R₁ ein ein- oder mehrkerniger basischer Heterocyclus mit einem oder mehreren Stickstoffatomen ist.

In einer bevorzugten Ausführungsform ist R₁ C₁ - C₆ -Alkylen, C₃ - C₆ -Cycloalkylen, C₃- C₆-Cycloalkenylen, C₂- C₆ -Alkenylen, C₆ - C₁₄ - Arylen oder ein Heterocyclus, die jeweils substituiert oder unsubstituiert sein können, und
R₂ und R₃ C₁ - C₁₀ -Alkyl, C₃ - C₆- Cycloalkyl, C₃ - C₆ -Cycloalkenyl, C₂ - C₁₀-Alkenyl, C₆ - C₁₄ - Aryl, oder ein Heterocyclus, die jeweils substituiert oder unsubstituiert sein können, oder Wasserstoff.

R₁ ist bevorzugt:
C₁ - C₅ -Alkylen, wie n-Butylen oder n-Pentylen, insbesondere C₁ - C₃ - Alkylen, wie Methylen, Ethylen, n-Propylen oder i-Propylen;
C₂ - C₅ Alkenylen, wie Butenylen oder Pentenylen, insbesondere C₂ - C₃ -Alkenylen, wie Ethenylen oder Propenylen;
C₃ - C₆-Cycloalkylen, wie Cyclopentylen oder Cyclohexylen, insbesondere C₃ - C₄-Cycloalkylen, wie Cyclopropylen oder Cyclobutylen;
C₃ - C₆-Cycloalkenylen, insbesondere C₅ - C₆-Cycloalkenylen, wie Cyclopentenylen oder Cyclohexenylen;
C₆ - C₁₀-Arylen, insbesondere Benzylen.

R₂ und R₃ sind bevorzugt:
C₁ - C₆ -Alkyl, wie n-Butyl, n-Pentyl, oder n-Hexyl, insbesondere C₁ - C₃ -Alkyl, wie Methyl, Ethyl, n-Propyl oder i-Propyl;
C₂ - C₆-Alkenyl, wie Butenyl, Pentenyl, insbesondere C₂ - C₃-Alkenyl, wie Ethenyl oder Propenyl;
C₃ - C₆-Cycloalkyl, wie Cyclopentyl oder Cyclohexyl, insbesondere C₃ - C₆-Cycloalkyl, wie Cyclopropyl oder Cyclobutyl;
C₃ - C₆-Cycloalkenyl, insbesondere C₅ - C₆-Cycloalkenyl, wie Cyclopentenyl oder Cyclohexenyl;
C₆ - C₁₀-Aryl, insbesondere Benzyl.

In einer bevorzugten Ausführungsform bilden R₄ und R₅ einen aromatischen Ring.

R₁, R₂ und/oder R₃ sind in bevorzugten Ausführungsformen substituiert durch:
Hydroxyl, Amino, -SO₃H, Halogen, C₁-C₄-Alkyl, C₂-C₄-Alkenyl, C₃-C₆-Cycloalkyl, C₃-C₆-Cycloalkenyl, C₁-C₄-Alkoxy, C₁-C₄-Alkoxy-C₁-C₄-alkylen, C₁-C₄-Alkylmercapto, C₁-C₄-Alkylmercapto-C₁-C₄-alkylen, Formyl, C₁-C₄-Alkoxycarbonyl, C₁-C₄-Alkoxycarbonyl-C₁-C₄-alkylen, Di-C₁-C₄-alkyl-amino, Di-C₁-C₄-alkylamino-C₁-C₄-alkylen, Di-C₁-C₄-alkylaminocarbonyl, Di-C₁-C₄-alkylaminocarbonyl-C₁-C₄-alkylen.

Weiterhin sind Gruppen der allgemeinen Formel (II) bevorzugt aus Gruppen der Formeln (VIII) und (IX) 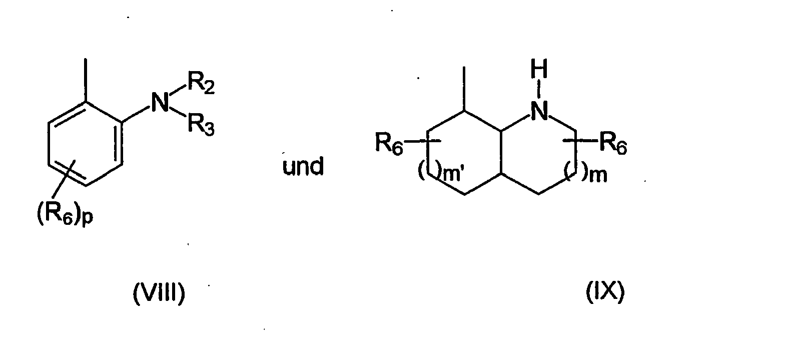 ausgewählt, wobei
- R₂, R₃: wie oben definiert,
- R₆: Alkyl, Cycloalkyl, Aryl oder Heteroaryl, die jeweils substituiert oder unsubstituiert sein können,
- p: 0 bis 4,
- m, m': 0 bis 2, insbesondere 1 sind.

Ferner können, falls p gleich 2 ist, zwei orthoständige Substituenten R₆ einen gegebenenfalls aromatischen Cyclus bilden.

R₆ ist in bevorzugten Ausführungsformen substituiert wie oben definiert für R₁, R₂ und/oder R₃.

Gruppen der allgemeinen Formel (III) sind bevorzugt aus Gruppen der Formeln (X) und (XI) 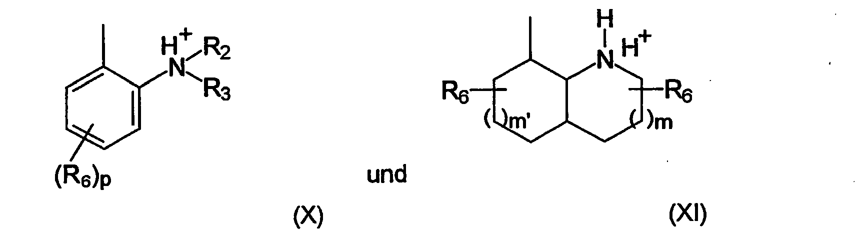 ausgewählt, wobei
- R₂, R₃, R₆, p, m, m': wie oben definiert sind.
Gruppen der allgemeinen Formel (IV) sind bevorzugt aus Gruppen der Formeln (XII) und (XIII) 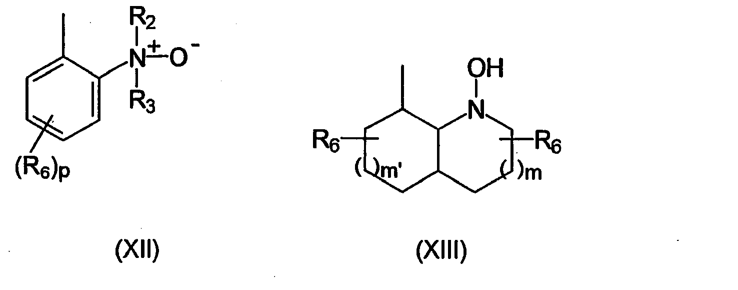 ausgewählt, wobei
- R₂, R₃, R₆, p, m, m': wie oben definiert sind.
Gruppen der allgemeinen Formel (V) sind bevorzugt aus den Formeln (XIV) und (XV) 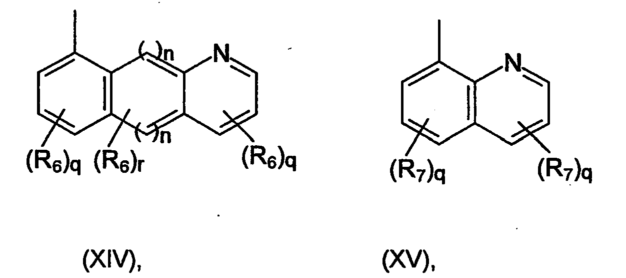 wobei
- R₆: wie oben,
- R₇: Alkyl, Cycloalkyl, Aryl oder Heteroaryl, die jeweils substituiert oder unsubstituiert sein können, Halogen, Sulfonyl,
- q: 0 bis 3,
- r: 0 bis 2, und
- n: 0 bis 2, insbesondere 1 sind, 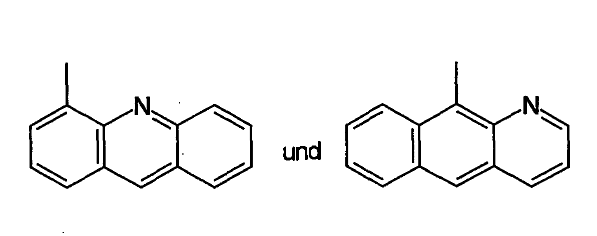
ausgewählt.

Ferner können, falls q oder r gleich 2 sind, zwei orthoständige Substituenten R₆ einen gegebenenfalls aromatischen Cyclus bilden.

Gruppen der allgemeinen Formel (VI) sind bevorzugt aus den Formeln (XVI) und (XVII) 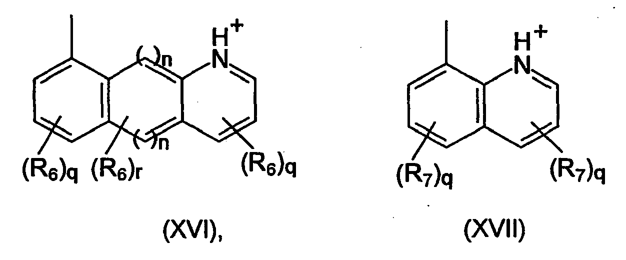 wobei
R₆, R₇, q, r, n wie oben definiert sind, 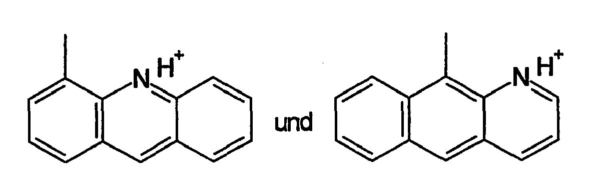 ausgewählt.
Gruppen der allgemeinen Formel (VII) sind bevorzugt aus den Formeln (XIII) und (XIX) 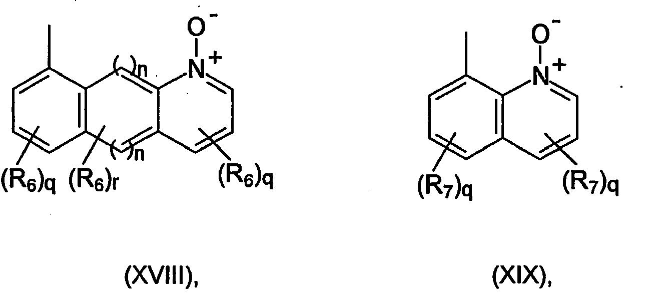 wobei
R₆, R₇, q, r, n
wie oben definiert sind, 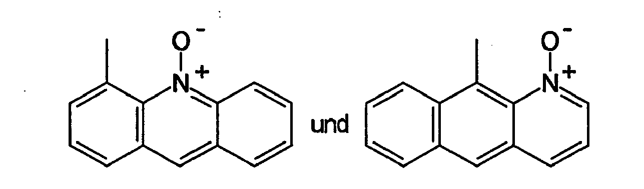 ausgewählt.

In einer weiteren bevorzugten Ausführungsform ist Y in der allgemeinen Formel (I) Chlor.

Besonders bevorzugt sind q und r = 0 in den Gruppen der Formeln (XIV), (XV), (XVI), (XVII), (XVIII) und (XIX).

Ganz besonders bevorzugt ist R in der allgemeinen Formel (I) eine Gruppe der Formel (XV) und q = 0.

Die Erfindung betrifft auch die Verwendung von Gallium(III)-Komplexen der allg. Formel (I) zur Herstellung eines Arzneimittels zur Behandlung von Melanomen.

Bei den Melanomen, die für Behandlung durch einen Galliumkomplex der allgemeinen Formel (I) geeignet sind, kann es sich um ein amelanotisches Melanom, ein Lentigo Maligna, ein Acral Lentiginous, ein Epitheloid-Zell Melanom, ein noduläres Melanom, ein Melanom in Verbindung mit Naevus, ein Melanom mit superficialem Spreading oder ein Spindelzell Melanom handeln. Auch Metastasen dieser Tumore in anderen Organen können durch einen Galliumkomplex der allg. Formel (I) behandelt werden.

Zur Behandlung der vorstehend genannten Krebserkrankungen wird der Gallium-Komplex der Fromel (I) besonders bevorzugt peroral, aber auch intravenös, intramuskulär, intraperitoneal oder subkutan verabreicht. Auch eine äußerliche oder lokale Applikation ist möglich. Bevorzugt ist die Verabreichung durch perorale Applikation.

Die erfindungsgemäße Verwendung von Gallium(III)-Komplexen kann in jeder geeigneten Formulierung durchgeführt werden unter der Voraussetzung, dass die Ausbildung bzw. Aufrechterhaltung von ausreichenden Wirkstoffpegeln gewährleistet ist. Das kann beispielsweise durch orale oder parenterale Gabe in geeigneten Dosen erreicht werden. Vorteilhafterweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragee, eine Kapsel, ein Suppositorium oder eine gemessene Volumenmenge eines Pulvers, eines Granulates, einer Lösung, einer Emulsion oder einer Suspension sein.

Unter "Einheitsdosis" im Sinne der vorliegenden Erfindung wird eine physikalisch bestimmte Einheit verstanden, die eine individuelle Menge des aktiven Bestandteils in Kombination mit einem pharmazeutischen Trägerstoff enthält und deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel der Einheitsdosis benötigt wird, ist die Einheitsdosis vorteilhafterweise teilbar, z.B. in Form einer Tablette mit Bruchkerbe.

Die erfindungsgemäßen Verwendung von Gallium(III)-Komplexen in einem geeigneten Arzneimittel kann, wenn sie in Einheitsdosen durchgeführt wird und für Applikationen z.B. am Menschen bestimmt ist, mit etwa 0,1 bis 3000 mg, bevorzugt 10 bis 2000 mg und insbesondere 30 bis 1500 mg Wirkstoff erfolgen. Der Wirkstoff kann einmalig verabreicht werden, aber auch kontinuierlich über einen längeren Zeitraum. Bei einer oralen Behandlung können ähnliche Dosierungen zur Anwendung kommen.

Die erfindungsgemäße therapeutische Verwendung von Gallium(III)-Komplexen in einem Arzneimittel kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter der zu behandelnden Individuen, der Art und Schwere der Erkrankung, der Art der Zubereitung und der Applikation der Arzneimittel, sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Es kann sich auch als zweckmäßig erweisen, die Arzneimittel nur einmalig oder im Abstand von mehreren Tagen zu verabreichen.

Die Festlegung der erforderlichen optimalen Dosierung und Applikationsart von Gallium(III)-Komplexen kann durch jeden Fachmann aufgrund seines Fachwissens erfolgen.

Die erfindungsgemäße Verwendung von Gallium(III)-Komplexen kann in Form von Arzneimitteln erfolgen, die in der Regel den Gallium(III)-Komplex und nichttoxische, pharmazeutisch verträgliche Arzneimittelträger umfassen, die als Zumischung oder Verdünnungsmittel, beispielsweise in fester, halbfester oder flüssiger Form oder als Umhüllungsmittel, beispielsweise in Form einer Kapsel, eines Tablettenüberzugs, eines Beutels oder eines anderen Behältnisses für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten Dragees, harte und weiche Kapseln, z.B. aus Gelatine, dispergierbare Pulver, Granulate, wässrige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Laktose; Granulierungs- und Verteilungsmittel, z.B. Maisstärke, Polyvinylpyrrolidon oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, dass er eine verzögerte Auflösung und Resorption der Arzneimittelzubereitung im Gastrointestinaltrakt bewirkt, so dass z.B. eine bessere Verträglichkeit, Protahierung oder Retardierung erreicht wird. Gelatinekapseln können den Arzneistoff vermischt mit einem festen, z.B. Calciumcarbonat oder Kaolin, oder einem öligen, z.B. Oliven-, Erdnuss-, oder Paraffinöl, Verdünnungsmittel enthalten.

Wässrige Suspensionen können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycatanol, Polyoxyethylensorbitolmonooleat oder Lecithin; Konservierungsmittel, z.B. Methyl- oder Propylhydroxybenzoate; Geschmacksmittel; Süßungsmittel, z.B. Saccharose, Laktose, Natriumcyclamat, Dextrose, Invertzuckersirup, enthalten.

Ölige Suspensionen können z.B. Erdnuss-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können bei der erfindungsgemäßen Verwendung von Gallium(III)-Komplexen in Mischung mit Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuss-, oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmittel enthalten.

Wässrige Lösungen können Konservierungsmittel, z.B. Methyl- oder Propylhydroxybenzoate; Verdickungsmittel; Geschmacksmittel; Süßungsmittel, z.B. Saccharose, Laktose, Natriumcyclamat, Dextrose, Invertzuckersirup, sowie Geschmacksmittel und Farbstoffe enthalten.

Zur parenteralen Anwendung der Arzneistoffe dienen steril injizierbare, wässrige Lösungen, isotonische Salzlösungen oder sonstige Lösungen.

Die Erfindung wird nachstehend anhand von Beispielen erläutert.

### Beispiele

Die Verbindung Tris-Hydroxychinolinolato-Gallium (III) wurde in der Zellkultur an den Melanom-Zellinien, die aus humanen Tumoren gewonnen worden sind, bezüglich ihrer zytotoxischen Aktivität untersucht.

Dabei zeigte die Verbindung eine hohe Aktivität im µmolaren Bereich:

| | |
|---|---|
| SK-MEL-5 | 0,76 |
| SK-MEL-28 | 35 |

## Patentansprüche

1. Verwendung einer Verbindung der allgemeinen Formel (I) 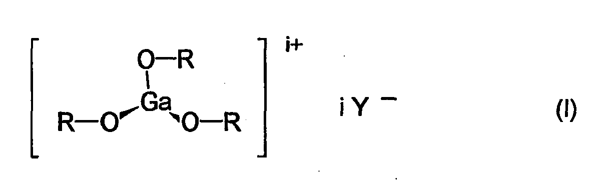 worin R
eine N-haltige Gruppe ist, ausgewählt aus einer Gruppe der allgemeinen Formel (II) bis (VII): 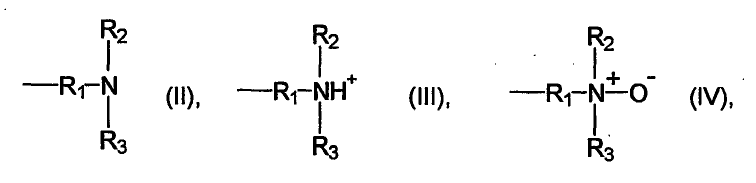 worin
R₁ C₁ - C₆ -Alkylen, C₃ - C₈ -Cycloalkylen, C₃ - C₈ -Cycloalkenylen, C₂ - C₆ -Alkenylen, ein ein- oder mehrkemiges gegebenenfalls aromatisches C₆ - C₁₄ -Ringsystem oder ein Heterocyclus, die jeweils substituiert oder unsubstituiert sein können, ist;
R₂ und R₃ C₁ - C₁₀ -Alkyl, C₃ - C₈- Cycloalkyl, C₃ - C₈- Cycloalkenyl, C₂ - C₁₀-Alkenyl, ein ein- oder mehrkemiges gegebenenfalls aromatisches C₆ - C₁₄ -Ringsystem, oder ein Heterocyclus, die jeweils substituiert oder unsubstituiert sein können, oder Wasserstoff ist;
und R₁ und R₂, oder R₁ und R₃, oder R₂ und R₃ einen Heterocyclus bilden können, der gegebenenfalls weitere Strickstoffatome enthalten kann; 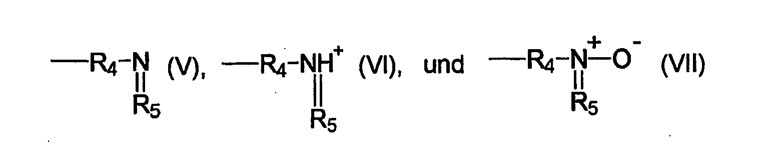 worin
R₄ die gleiche Bedeutung wie R₁ besitzt, R₅ die gleiche Bedeutung wie R₂ besitzt, und
R₄ und R₅ zusammen mit N ein gegebenenfalls aromatisches Ringsystem bilden können, das weitere Stickstoffatome enthalten kann;
oder
i eine ganze Zahl von 0 bis 3 ist und der Summe der N-halfigen Gruppen der Formeln (III) und/oder (VI) entspricht;
Y ein Halogen, Pseudohalogen, HCO₃ oder R'COO, worin R' C₁ - C₆ -Alkyl, C₂ - C₆ -Alkenyl, C₃ - C₆ -Cycloalkyl, C₃ - C₆ -Cycloalkenyl, Aryl, die jeweils substituiert oder unsubstituiert sein können, und/oder ein physiologisch verträgliches Anion ist; und
physiologisch verträgliche Additionssalze davon,
zur Herstellung eines Arzneimittels zur Behandlung von Melanomen.

2. Verwendung einer Verbindung nach Anspruch 1, worin R 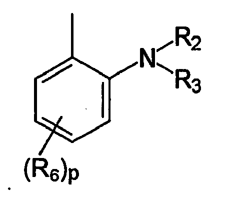 ist, wobei
R₆ Alkyl, Cycloalkyl, Aryl oder Heteroaryl, und
p 0 bis 4 sind.

3. Verwendung einer Verbindung nach Anspruch 1, worin R 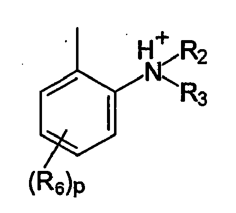 ist, wobei
R₆ Alkyl, Cycloalkyl, Aryl oder Heteroaryl, und
p 0 bis 4 sind.

4. Verwendung einer Verbindung nach Anspruch 1, worin R 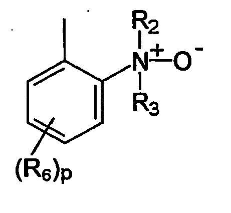 ist, wobei
R₆ Alkyl, Cycloalkyl, Aryl oder Heteroaryl, und
p 0 bis 4 sind.

5. Verwendung einer Verbindung nach einem der Ansprüche 2 bis 4, wobei p = 0 ist.

6. Verwendung einer Verbindung nach Anspruch 1, worin R 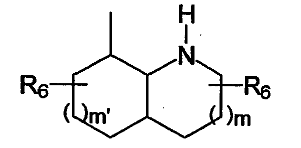 ist, wobei
R₆ Alkyl, Cycloalkyl, Aryl oder Heteroaryl, und
m, m' 0 bis 2 sind.

7. Verwendung einer Verbindung nach Anspruch 1, worin R 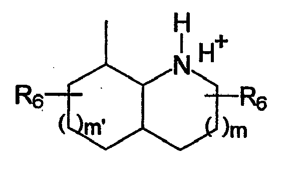 ist, wobei
R₆ Alkyl, Cycloalkyl, Aryl oder Heteroaryl, und
m, m' 0 bis 2 sind.

8. Verwendung einer Verbindung nach Anspruch 1, worin R 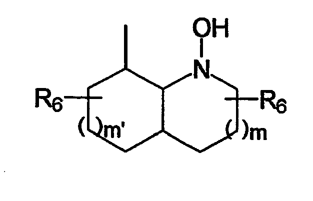 ist, wobei
R₆ Alkyl, Cycloalkyl, Aryl oder Heteroaryl, und
m, m' 0 bis 2 sind.

9. Verwendung einer Verbindung nach einem der Ansprüche 6 bis 8, wobei m und m' 1 sind.

10. Verwendung einer Verbindung nach Anspruch 1, worin R 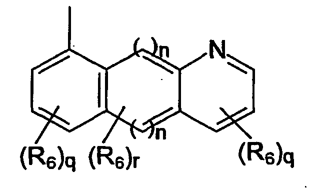 ist, wobei
R₆ Alkyl, Cycloalkyl, Aryl oder Heteroaryl,
q 0 bis 3,
r 0 bis 2, und
n 0 bis 2 sind.

11. Verwendung einer Verbindung nach Anspruch 1, worin R 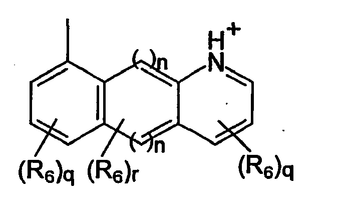 ist, wobei
R₆ Alkyl, Cycloalkyl, Aryl oder Heteroaryl,
q 0 bis 3,
r 0 bis 2, und
n 0 bis 2 sind.

12. Verwendung einer Verbindung nach Anspruch 1, worin R 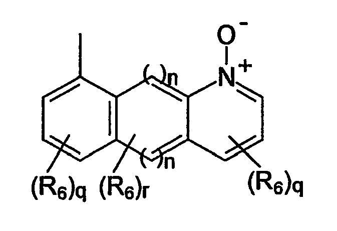 ist, wobei
R₆ Alkyl, Cycloalkyl, Aryl oder Heteroaryl,
q 0 bis 3,
r 0 bis 2, und
n 0 bis 2 sind.

13. Verwendung einer Verbindung nach einem der Ansprüche 10 bis 12, wobei n gleich 1 ist.

14. Verwendung einer Verbindung nach Anspruch 1, worin R 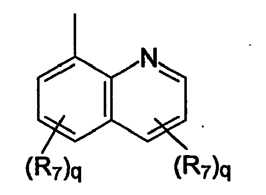 ist, wobei
R₇ Alkyl, Cycloalkyl, Aryl oder Heteroaryl, die jeweils substituiert oder unsubstituiert sein können, Halogen, Sulfonyl, und
q 0 bis 3 ist.

15. Verwendung einer Verbindung nach Anspruch 1, worin R 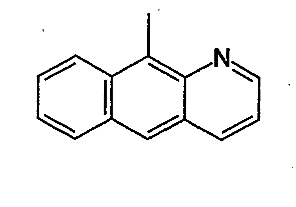 ist.

16. Verwendung einer Verbindung nach Anspruch 1, worin R 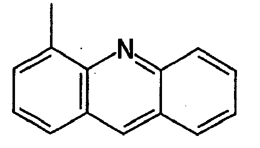 ist.

17. Verwendung einer Verbindung nach Anspruch 1, worin R 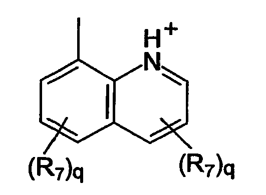 ist, wobei
R₇ Alkyl, Cycloalkyl, Aryl oder Heteroaryl, die jeweils substituiert oder unsubstituiert sein können, Halogen, Sulfonyl, und
q 0 bis 3 ist.

18. Verwendung einer Verbindung nach Anspruch 1, worin R 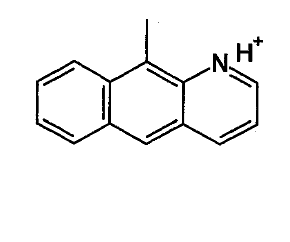 ist.

19. Verwendung einer Verbindung nach Anspruch 1, worin R 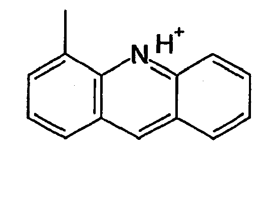 ist.

20. Verwendung einer Verbindung nach Anspruch 1, worin R 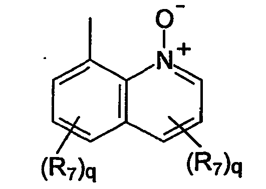 ist, wobei
R₇ Alkyl, Cycloalkyl, Aryl oder Heteroaryl, die jeweils substituiert oder unsubstituiert sein können, Halogen, Sulfonyl, und
q 0 bis 3 ist.

21. Verwendung einer Verbindung nach Anspruch 1, worin R 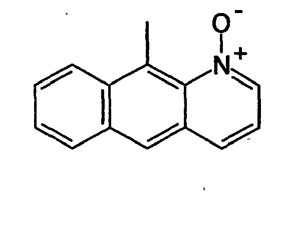 ist.

22. Verwendung einer Verbindung nach Anspruch 1, worin R 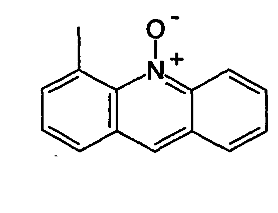 ist.

23. Verwendung einer Verbindung nach mindestens einem der Ansprüche 1 bis 22, worin Y Chlor ist.

24. Verwendung einer Verbindung nach einem der Ansprüche 14, 17 oder 20, wobei q gleich 0 ist.

25. Verwendung von Tris-Hydroxychinolinolato-Gailium (III) zur Behandlung von Melanomen.

26. Verwendung einer Verbindung nach einem der Ansprüche 1-25 zur Behandlung von Metastasen eines Melanoms.

## Claims

1. Use of a compound of the general formula (I) 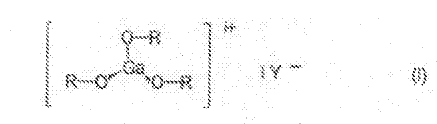 wherein
R is an N-containing group selected from the group with the general formulae (II) to (VII): 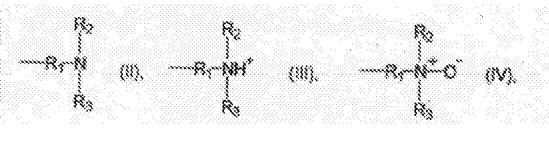 wherein
R₁ is C₁-C₆ alkylene, C₃-C₈ cycloalkylene, C₃-C₈ cycloalkenylene, C₂-C₆ alkenylene, a mono- or polynuclear, possibly aromatic, C₆-C₁₄ ring system or a heterocycle, which can respectively be substituted or unsubstituted;
R₂ and R₃ are C₁-C₁₀ alkyl, C₃-C₈cycloalkyl, C₃-C₈ cycloalkenyl, C₂-C₁₀alkenyl, a mono- or polynuclear, possibly aromatic, C₆-C₁₄ ring system or a heterocycle, which can respectively be substituted or unsubstituted, or hydrogen;
and R₁ and R₂, or R₁ and R₃, or R₂ and R₃ can form a heterocycle that can possibly contain further nitrogen atoms; 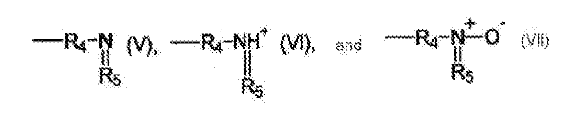 wherein
R₄ has the same meaning as R₁, R₅ has the same meaning as R₂, and
R₄ and R₅ together with N can form a possibly aromatic ring system that can contain further nitrogen atoms;
or
i corresponds to a whole number from 0 to 3 and the sum of the N-containing groups of formulae (III) and/or (VI);
Y is a halogen, pseudohalogen, HCO₃ or R'COO, wherein R' is C₁-C₆ alkyl, C₂-C₆ alkenyl, C₃-C₆ cycloalkyl, C₃-C₆ cycloalkenyl, aryl, which can respectively be substituted or unsubstituted, and/or a physiologically compatible anion; and physiologically compatible addition salts thereof,
for the production of a medication for the treatment of melanomas.

2. Use of a compound according to claim 1, wherein R is 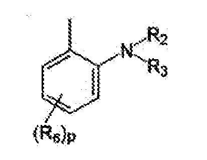 wherein
R₆ is alkyl, cycloalkyl, aryl or heteroaryl, and
p is 0 to 4.

3. Use of a compound according to claim 1, wherein R is 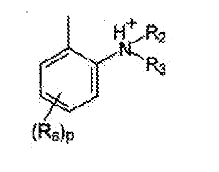 wherein
R₆ is alkyl, cycloalkyl, aryl or heteroaryl, and
p is 0 to 4.

4. Use of a compound according to claim 1, wherein R is 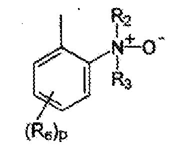 wherein
R₆ is alkyl, cycloalkyl, aryl or heteroaryl, and
p is 0 to 4.

5. Use of a compound according to one of claims 2 to 4, wherein p=0.

6. Use of a compound according to claim 1, wherein R is 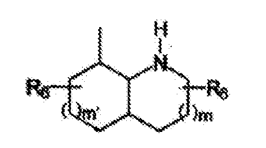 wherein
R₆ is alkyl, cycloalkyl, aryl or heteroaryl, and
m, m' is 0 to 2.

7. Use of a compound according to claim 1, wherein R is 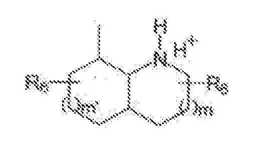 wherein
R₆ is alkyl, cycloalkyl, aryl or heteroaryl, and
m, m' is 0 to 2.

8. Use of a compound according to claim 1, wherein R is 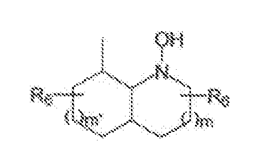 wherein
R₆ is alkyl, cycloalkyl, aryl or heteroaryl, and
m, m' is 0 to 2.

9. Use of a compound according to one of claims 6 to 8, wherein m and m' are 1.

10. Use of a compound according to claim 1, wherein R is 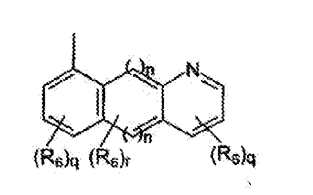 wherein
R₆ is alkyl, cycloalkyl, aryl or heteroaryl,
q 0 to 3,
r 0 to 2, and
n is 0 to 2.

11. Use of a compound according to claim 1, wherein R is 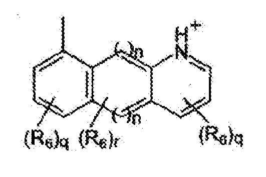 wherein
R₆ is alkyl, cycloalkyl, aryl or heteroaryl,
q 0 to 3,
r 0 to 2, and
n is 0 to 2.

12. Use of a compound according to claim 1, wherein R is 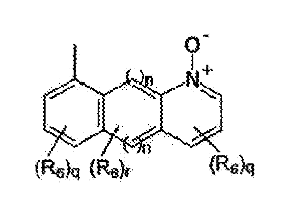 wherein
R₆ is alkyl, cycloalkyl, aryl or heteroaryl,
q 0 to 3,
r 0 to 2, and
n is 0 to 2.

13. Use of a compound according to one of claims 10 to 12, wherein n is equal to 1.

14. Use of a compound according to claim 1, wherein R is 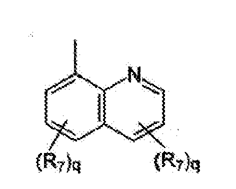 wherein
R₇ is alkyl, cycloalkyl, aryl or heteroaryl, which can respectively be substituted or unsubstituted, halogen, sulphonyl, and
q is 0 to 3.

15. Use of a compound according to claim 1, wherein R is

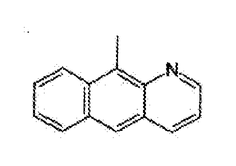

16. Use of a compound according to claim 1, wherein R is

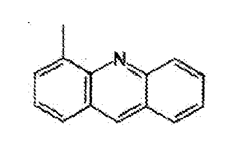

17. Use of a compound according to claim 1, wherein R is 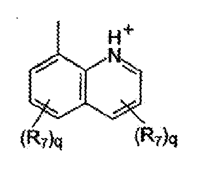 wherein
R₇ is alkyl, cycloalkyl, aryl or heteroaryl, which can respectively be substituted or unsubstituted, halogen, sulphonyl, and
q is 0 to 3.

18. Use of a compound according to claim 1, wherein R is

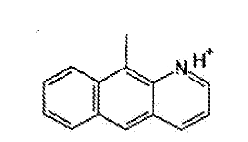

19. Use of a compound according to claim 1, wherein R is

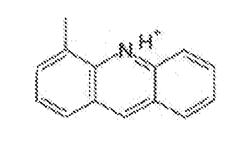

20. Use of a compound according to claim 1, wherein R is 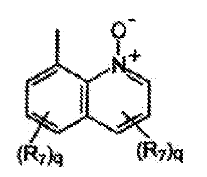 wherein
R₇ is alkyl, cycloalkyl, aryl or heteroaryl, which can respectively be substituted or unsubstituted, halogen, sulphonyl, and
q is 0 to 3.

21. Use of a compound according to claim 1, wherein R is

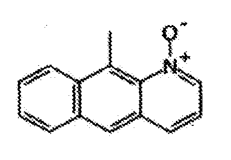

22. Use of a compound according to claim 1, wherein R is

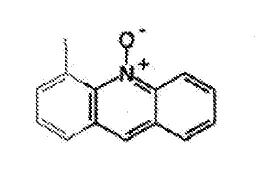

23. Use of a compound according to at least one of claims 1 to 22, wherein Y is chlorine.

24. Use of a compound according to at least one of claims 14, 17 or 20, wherein q is equal to 0.

25. Use of tris-hydroxyquinolinolato-gallium (III) for the treatment of melanomas.

26. Use of a compound according to one of claims 1 to 25 for the treatment of metastases of a melanoma.

## Revendications

1. Utilisation d'une liaison de formule générale (I) 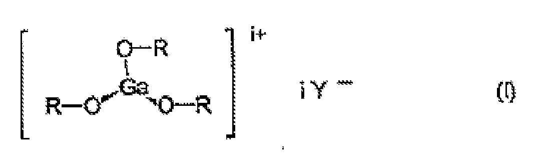 dans laquelle R est un groupe contenant N, choisi parmi un groupe de la formule générale (II) à (VII) : 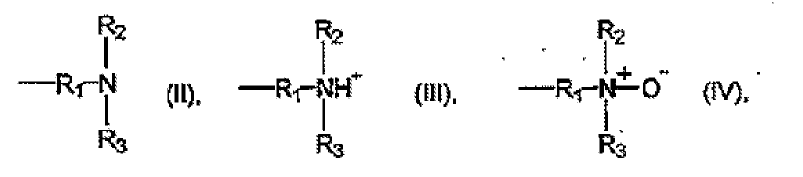 dans laquelle
R₁ est un C₁-C₆-alkylène, un C₃-C₈-cycloalkylène, un C₃-C₈-cycloalkénylène, un C₂-C₆-alkénylène, un système cyclique le cas échéant aromatique C₆-C₁₄ à un ou plusieurs noyaux ou un hétérocycle, pouvant respectivement être substitués ou non substitués ;
R₂ et R₃ sont un C₁-C₁₀-alkyle, un C₃-C₈-cycloalkyle, un C₃-C₈-cycloalkényle, un C₂-C₁₀-alkényle, un système cyclique le cas échéant aromatique C₆-C₁₄ à un ou plusieurs noyaux ou un hétérocycle pouvant respectivement être substitués ou non substitués, ou bien de l'hydrogène ;
et R₁ et R₂, ou R₁ et R₃, ou R₂ et R₃ peuvent former un hétérocycle pouvant le cas échéant contenir d'autres atomes d'azote ; 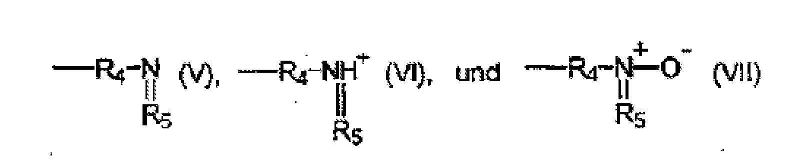 dans laquelle
R₄ a la même signification que R₁, R₅ a la même signification que R₂, et R₄ et R₅ peuvent former avec N un système cyclique le cas échéant aromatique pouvant contenir d'autres atomes d'azote ;
ou
i est un nombre entier compris entre 0 et 3 et correspond à la somme des groupes contenant N des formules (III) et/ou (VI) ;
Y est un halogène, un pseudohalogène, HCO₃ ou R'COO, R' étant un C₁-C₆-alkyle, un C₂-C₆-alkényle, un C₃-C₆-cycloalkyle, un C₃-C₆-cycloalkényle, un aryle, pouvant respectivement être substitués ou non substitués, et/ou est un anion physiologiquement tolérable, et des sels d'addition physiologiquement tolérables de ces derniers,
pour la fabrication d'un médicament destiné au traitement des mélanomes.

2. Utilisation d'une liaison selon la revendication 1, dans laquelle R est 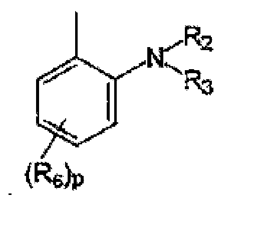 où
R₆ est un alkyle, un cycloalkyle, un aryle ou un hétéroaryle, et
p est compris entre 0 et 4.

3. Utilisation d'une liaison selon la revendication 1, dans laquelle R est 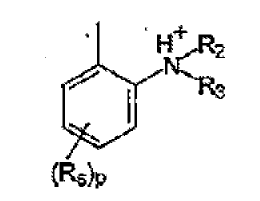 où
R₆ est un alkyle, un cycloalkyle, un aryle ou un hétéroaryle, et
p est compris entre 0 et 4.

4. Utilisation d'une liaison selon la revendication 1, dans laquelle R est 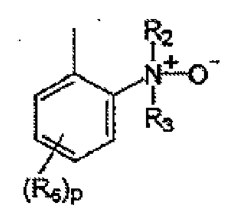 où
R₆ est un alkyle, un cycloalkyle, un aryle ou un hétéroaryle, et
p est compris entre 0 et 4.

5. Utilisation d'une liaison selon l'une des revendications 2 à 4, dans laquelle p = 0.

6. Utilisation d'une liaison selon la revendication 1, dans laquelle R est 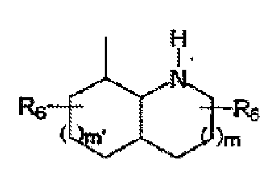 où
R₆ est un alkyle, un cycloalkyle, un aryle ou un hétéroaryle, et
m, m' sont compris entre 0 et 2.

7. Utilisation d'une liaison selon la revendication 1, dans laquelle R est 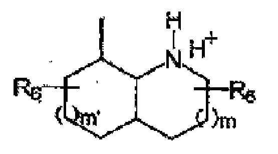 où
R₆ est un alkyle, un cycloalkyle, un aryle ou un hétéroaryle, et
m, m' sont compris entre 0 et 2.

8. Utilisation d'une liaison selon la revendication 1, dans laquelle R est 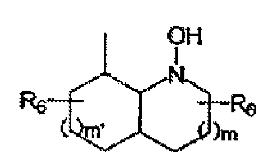 où
R₆ est un alkyle, un cycloalkyle, un aryle ou un hétéroaryle, et
m, m' sont compris entre 0 et 2.

9. Utilisation d'une liaison selon l'une des revendications 6 à 8, dans laquelle m et m' sont égaux à 1.

10. Utilisation d'une liaison selon la revendication 1, dans laquelle R est 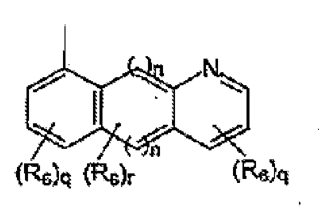 où
R₆ est un alkyle, un cycloalkyle, un aryle ou un hétéroaryle,
q est compris entre 0 et 3,
r est compris entre 0 et 2, et
n est compris entre 0 et 2.

11. Utilisation d'une liaison selon la revendication 1, dans laquelle R est 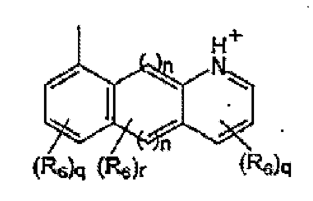 où
R₆ est un alkyle, un cycloalkyle, un aryle ou un hétéroaryle,
q est compris entre 0 et 3,
r est compris entre 0 et 2, et
n est compris entre 0 et 2.

12. Utilisation d'une liaison selon la revendication 1, dans laquelle R est 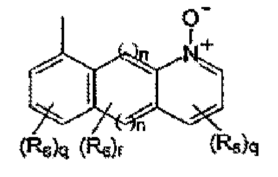 où
R₆ est un alkyle, un cycloalkyle, un aryle ou un hétéroaryle,
q est compris entre 0 et 3,
r est compris entre 0 et 2, et
n est compris entre 0 et 2.

13. Utilisation d'une liaison selon l'une des revendications 10 à 12, dans laquelle n est égal à 1.

14. Utilisation d'une liaison selon la revendication 1, dans laquelle R est 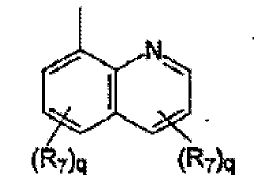 où
R₇ est un alkyle, un cycloalkyle, un aryle ou un hétéroaryle, pouvant respectivement être substitués ou non substitués, un halogène, un sulfonyle, et
q est compris entre 0 et 3.

15. Utilisation d'une liaison selon la revendication 1, dans laquelle R est

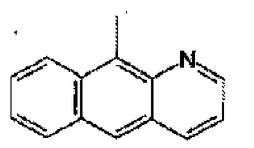

16. Utilisation d'une liaison selon la revendication 1, dans laquelle R est

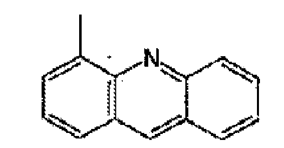

17. Utilisation d'une liaison selon la revendication 1, dans laquelle R est 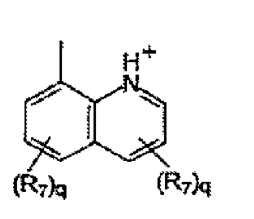 où
R₇ est un alkyle, un cycloalkyle, un aryle ou un hétéroaryle, pouvant respectivement être substitués ou non substitués, un halogène, un sulfonyle, et
q est compris entre 0 et 3.

18. Utilisation d'une liaison selon la revendication 1, dans laquelle R est

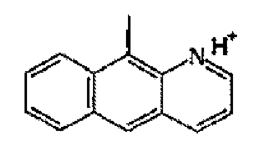

19. Utilisation d'une liaison selon la revendication 1, dans laquelle R est

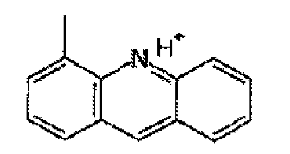

20. Utilisation d'une liaison selon la revendication 1, dans laquelle R est 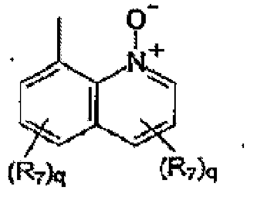 où R₇ est un alkyle, un cycloalkyle, un aryle ou un hétéroaryle, pouvant respectivement être substitués ou non substitués, un halogène, un sulfonyle, et
q est compris entre 0 et 3.

21. Utilisation d'une liaison selon la revendication 1, dans laquelle R est

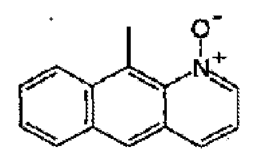

22. Utilisation d'une liaison selon la revendication 1, dans laquelle R est

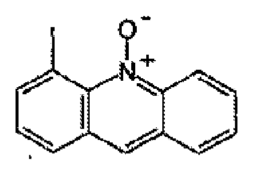

23. Utilisation d'une liaison selon au moins l'une des revendications 1 à 22, dans laquelle Y est du chlore.

24. Utilisation d'une liaison selon l'une des revendications 14, 17 ou 20, dans laquelle q est égal à 0.

25. Utilisation de Tris-hydroxychinolinolato-gallium (III) pour le traitement de mélanomes.

26. Utilisation d'une liaison selon l'une des revendications 1 à 25 pour le traitement de métastases d'un mélanome.
